# EUROPEAN PATENT APPLICATION

(11) **EP 4 589 298 A1**
(43) Date of publication of application: **23.07.2025**
(21) Application number: 24152941.1
(22) Date of filing: 19.01.2024
(51) Int. Cl.: G01N 33/532, G01N 33/533, G01N 33/535

(54) **MARKER AND METHOD FOR ANALYSING A BIOLOGICAL SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

In a first aspect a marker (100, 404, 500) for analysing a biological sample with a plurality of target analytes (107, 113, 406, 506) is provided. The marker (100, 404, 500) comprises a first marker part (102) comprising a first affinity reagent (106, 400, 502) and a first label, the first label comprising a first polymeric backbone (108) attached to the first affinity reagent (106, 400, 502) and a plurality of first labelling moiety parts (110) attached to the first polymeric backbone (108), and a second marker part (104) comprising a second affinity reagent (112, 402, 504) and a second label, the second label comprising a second polymeric backbone (114) attached to the second affinity reagent (112, 402, 504) and a plurality of second labelling moiety parts (116) attached to the second polymeric backbone (114). The first labelling moiety parts (110) and the second labelling moiety parts (116) are configured to bind to the respective other one (110, 116) to form labelling moieties (118). The first affinity reagent and the second affinity reagent are each configured to bind specifically to one of the target analytes of the biological sample. In a further aspect, a method for analysing a biological sample is provided.

## Description

### Technical field

The invention relates to a marker for analysing a biological sample with a plurality of target analytes. In further aspects, a labelling kit and a method for analysing a biological sample are provided.

### Background

Labels and markers are frequently used when analysing biological samples, for example in fluorescent microscopy. Markers for fluorescent microscopy generally comprise affinity reagents, such as antibodies, and labels attached to the affinity reagents, in order to enable specifically attaching the labels- in particular via the affinity reagents - to a target analyte in a biological sample. This allows the identification and/or localisation of the target analyte in the biological sample.

The various applications of markers include multiplexing approaches, which require large sets of distinguishable markers, whilst detecting analytes that only occur in small quantities requires markers with bright labels. It is of constant interest to provide markers and labels for microscopy applications that allow flexible use and high reliability when detecting and identifying target analytes.

### Summary

It is an object to provide markers for analysing a biological sample that are flexible and reliable in use as well as a respective method for analysing biological samples.

The aforementioned object is achieved by the subject-matter of the independent claims. Advantageous embodiments are defined in the dependent claims and the following description.

In a first aspect, a marker for analysing a biological sample with a plurality of target analytes is provided. The marker comprises a first marker part and a second marker part. The first marker part comprises a first affinity reagent and a first label, the first label comprises a first polymeric backbone attached to the first affinity reagent and a plurality of first labelling moiety parts attached to the first polymeric backbone. The second marker part comprises a second affinity reagent and a second label, the second label comprises a second polymeric backbone attached to the second affinity reagent and a plurality of second labelling moiety parts attached to the second polymeric backbone. The first labelling moiety parts and the second labelling moiety parts are configured to bind to the respective other one to form labelling moieties. The first affinity reagent and the second affinity reagent are each configured to bind specifically to one of the target analytes of the biological sample.

In case the marker parts bind to entirely different target analytes, the marker enables determining the proximity or the distance between these different target analytes. In case the marker parts bind to the same target analyte but different areas or epitopes of that target analyte, the marker enables determining the presence or location of that target analytes with high precision or reliability. In particular, the closer the first marker part is to the second marker part, the more labelling moieties may form from the first labelling moiety parts and second labelling moiety parts. The proximity of the first marker part and the second marker part relates to the proximity of the first affinity reagent and the second affinity reagent. A ratio of formed labelling moieties to first and second labelling moiety parts may be detected optically by means of a microscope, for example.

Thus, the first affinity reagent may be specific to a first target analyte of the biological sample and the second affinity reagent may be specific to a second target analyte of the biological sample, for example. In a further example, the first affinity reagent may be specific to a first epitope of the first target analyte of the biological sample and the second affinity reagent may be specific to a second epitope of the first target analyte. Thus, the marker parts may bind to the same target analyte at the same time. This enables flexible use of the marker.

For example, the marker may be applied in a robust assay for proximity of at least two target analytes, which may be for example two distinct proteins, in which case the proximity may be taken as a proxy for interaction between the molecules, or they may be for example two epitopes on the same target protein. The latter is particularly interesting in cases, when the specificity of the assay shall be strongly improved. Owing to the cross-reactivity inherent to most if not all affinity reagents probing the same target with two affinity reagents that become part of one marker is a good strategy to render the assay more specific and reduce false-positive rate. This is not only of interest in life science research for multi-plex assays, but also for diagnostic tests which should discern closely related proteins from pathogenic and non-pathogenic strains for example.

The first and second labelling moiety parts preferably bind to the respective other one when in immediate proximity to each other, in particular. Preferably, each first and second labelling moiety part binds to a single one of the respective other one.

For example, the first and/or second affinity reagents may be one of an antibody, nanobody, an antibody-fragment or a nanobody-fragment, an aptamer, an aptabody, a polymeric binder, a drug, a toxin, a drug-like molecule, or another small molecule so as long as it is configured to bind a target analyte with high affinity and specificity. The aforementioned affinity reagents may be used to detect the presence of proteins for example.

Preferably, the first labelling moiety parts and the second labelling moiety parts are configured to bind to each other, or react with each other, under a catalytic condition, in particular, only under the catalytic condition. This enables control over the binding of the marker parts to each other and therefore control over the generation of the marker, preferably after the introduction of the first labelling moiety parts and the second labelling moiety parts into the biological sample. In particular, the first labelling moiety parts and the second moiety parts are configured to form the labelling moieties under the catalytic condition. The catalytic condition may be an environmental condition. For example, the catalytic condition maybe characterised by the presence of a particular catalyst, such as (UV-)light or copper ions. Thus, the first labelling moiety parts and the second moiety parts may react with each other. In particular, this may form a covalent bond between one of the first labelling moiety parts and one of the second labelling moiety parts.

Preferably, the first label is attached to the first affinity reagent by a first cleavable linker and/or the second label is attached to the second affinity reagent by a second cleavable linker. This enables easy inactivation of the marker. In particular, the cleavable linker may be configured to be specifically cleaved by a cleaving agent. In one example, the first cleavable linker and/or the second cleavable linker are part of the respective polymeric backbone.

Preferably, the first polymeric backbone and/or the second polymeric backbone comprises at least one nucleic acid. In a particular embodiment, the polymeric backbone is a nucleic acid.

Preferably, the first polymeric backbone is attached to the first affinity reagent by hybridisation and/or the second polymeric backbone is attached to the second affinity reagent by hybridisation. This enables easy assembly of the marker. For example, the respective affinity reagent may comprise an oligonucleotide with a sequence complementary to a part of the respective backbone. The aforementioned cleavable linkers may be part of the oligonucleotide of the affinity reagent.

Preferably, the first polymeric backbone extends away from the first affinity reagent in a first direction and the first labelling moiety parts are arranged on the first polymeric backbone along the first direction and/or the second polymeric backbone extends away from the second affinity reagent in a second direction and the second labelling moiety parts are arranged on the second polymeric backbone along the second direction. This enables a staggered formation of the labelling moieties according to the proximity or distance of the first and second affinity reagents to each other. The polymeric backbones extend in the respective direction in an essentially linear fashion, in particular. Preferably, the labelling moiety parts are arranged on the respective polymeric backbone next to each other along the respective direction, in particular, in a single file.

Preferably, each first labelling moiety part is equally spaced from any adjacent first labelling moiety part along the first polymeric backbone and/or each second labelling moiety part is equally spaced from any adjacent second labelling moiety part along the second polymeric backbone. This enables a precise correlation between the number of labelling moieties formed from the first and second labelling moiety parts and the proximity or distance between the first and second affinity reagents.

Preferably, the labelling moieties are optically detectable. This enables efficient detection of the labelling moieties, for example, by means of a microscope. In particular, only the labelling moieties are optically detectable. Thus, the first and second labelling moiety parts may not be optically detectable, for example. For example, the labelling moieties may be fluorophores.

Preferably, the first and second labelling moiety parts may bind to the respective other one by a click reaction, in particular, in order to generate optically detectable labelling moieties. Examples of fluorogenic click reactions include for example: (1) Copper-catalysed azide-alkyne cycloaddition (CuAAC) involving azides and alkynes, (2) Strain-promoted azide-alkyne cycloaddition (SPAAC), (3) Diels-Alder click reaction, (4) Inverse-electron-demand Diels-Alder reaction (iEDDA), (5) Staudinger ligation, (6) Thiol-ene reaction, and (7) strain-release click reactions. Further the following specific examples of suitable reactions are provided: one example is based on coumarin derivatives, wherein a 7-alkynyl group *"in introduced to quench the fluorescence of the coumaring core"* as reviewed in Droumaguet et al. 2010 (Chem. Soc. Rev., 2010, 39, 1233-1239). In this case the reaction with an azide group and formation of the triazole ring restores the fluorescence. As this is a copper-dependent CuAAC reaction, it is well suitable for the methods described in this document, for example the copper may be the catalyst providing the catalytic condition. It is important to note, that in this sense the azide group may be sufficient to serve as the second labelling moiety part. Another example *"developed a series of fluorogenic 3-azidocoumarins).",* which are non-fluorescent *"because of the electron-rich a-nitrogen of the azido group"* as reviewed in Droumaguet et al. 2010 (Chem. Soc. Rev., 2010, 39, 1233-1239). Furthermore, the the example *"synthesized non-fluorescent 1,8-naphthalimides with an alkyne or azide of the 4-position".* Another example are azido-anthracenes, which can be used for fluorogenic CuAAC with alkynes. Likewise, Xie and Wang generated non-fluorescent BODIPY *"with an azido-group of the 3-position of the pyrrole ring",* which undergo fluorogenic CuAAC with alkynes.

Preferably, the first labelling moiety parts and/or the second labelling moiety parts are optically detectable. In a particular embodiment, the first labelling moiety parts and the second labelling moiety parts may be optically detectable and the subsequently formed labelling moiety is not optically detectable. This enables determining whether or not the first marker part and the second marker part are in proximity. For example, the first labelling moiety parts maybe fluorescent dyes and the second labelling moiety parts maybe quenchers. Suitable quenchers may for example be: DABCYL, QSY-35, BHQ-1, QSY-7, QSY-9, BHQ-2, QSY-21, BHQ-3.

Preferably, the first labelling moiety parts and the second labelling moiety parts differ in their optically detectable properties from the optically detectable properties of the labelling moiety. This enables detecting the first and second marker parts individually and therefore distinguishing between the first and second marker parts. The optically detectable properties may include excitation or emission wavelength, or emission lifetime, in particular, if at least one of the first labelling moiety parts, the second labelling moiety parts and the labelling moieties comprise a fluorophore. In a particular embodiment, the first labelling moiety part and the second labelling moiety part may be configured to enable a non-radiative energy transfer between each other. For example, the first labelling moiety part and the second labelling moiety part may be a fluorescence resonance energy transfer pair. More specifically, the first labelling moiety part may be for example ATTO565-DBCO and the second labelling moiety part maybe for example ATTO633-Azide, which are configured to react with each other to form an ATTO565-633-Triazole as described on the manufacturer's website (ATTO-TEC GmbH, Siegen, Germany; Link: https://www.atto-tec.com/fluoreszenz/foerster-resonanz-energie-tansfer-fret/?language=de). In this example the ATTO565-DBCO will spontaneously react with the ATTO633-Azide to form a covalently conjugated FRET pair, exhibiting "almost complete transfer of the excitation energy". For the marker and methods proposed in this disclosure, it is desirable to avoid spontaneous reaction and to choose chemistries like Cu(I)-catalyzed alkyne-azide cycloaddition (CuCAAC) or other coupling chemistries that can be triggered by addition of a catalyst, pH change, UV-light or another external stimulus that creates a second, or catalytic, condition under which the first labelling moiety part and the second labelling moiety part react. Examples of FRET pairs, which may be used for such markers and methods include:
ATTO 425 - ATTO 520
ATTO 488 - ATTO 550, ATTO 565, ATTO 647N, ATTO 655
ATTO 520 - ATTO 647N
ATTO 532 - ATTO 647N, ATTO 655
ATTO 550 - ATTO 590, ATTO 647N
ATTO 565 - ATTO 590, ATTO 647N
ATTO 590 - ATTO 620, ATTO 647N, ATTO 680
ATTO 620 - ATTO 680

In another aspect, a method for analysing a biological sample is provided. The method comprises the steps of: Introducing at least one marker, in particular a plurality of markers, as described above into the biological sample and generating an optical readout of the biological sample with the marker. In particular, the biological sample comprises a plurality of target analytes. The at least one marker, in particular the respective affinity reagent, may be specific to one of the target analytes of the biological sample. The marker may be generated prior to introduction to the biological sample. Alternatively, the marker parts, specifically, the first and second marker parts may be introduced into the biological sample simultaneously or separately and subsequently.

Preferably, during or prior to the step of introducing the marker, a non-catalytic condition is applied to the biological sample. This avoids aggregation of markers in solution and enables efficient penetration of the marker into the biological sample. In particular, the step of introducing the marker is carried out under the non-catalytic condition. Preferably, the introduced marker only forms under the catalytic condition by the binding of the first and second labelling moiety parts to each other.

Preferably, after the step of introducing the marker, a catalytic condition is applied to the biological sample. This enables generating the marker in the biological sample, in particular the binding of the first and second labelling moiety parts to each other.

As described above, different configurations of the marker, method, or assay may be used for distinct applications, for example:
- **Fluorogenic assay:** In this case the first and second labelling moiety parts are essentially non-fluorescent and/or not optically detectable. Only after the marker parts are bound to their targets and after a preferred second condition (catalytic condition) is applied, the first labelling moiety parts and the second labelling moiety parts react to form the labelling moieties like for example a fluorescent dye, which can be optically detected. The same applies to non-fluorescent but otherwise optically detectable labelling moieties like for example polyynes (Raman imaging) or luminescent/phosphorescent molecules.
- **Quenching assay:** In this case one of the first labelling moiety parts and the second labelling moiety parts are fluorescent and/or optically detectable and the other labelling moiety part is a quencher. Only after the marker parts are bound to their targets and after a preferred second condition (catalytic condition) is applied the first labelling moiety parts and the second labelling moiety parts react to form the labelling moieties. In this case one of the educts is detectable optically and the product is not (inverted detection).
- **FRET assay:** In this case one of the first labelling moiety parts and the second labelling moiety parts are fluorescent and/or optically detectable and the labelling moiety parts are a FRET pair. Only after the marker parts are bound to their targets and after a preferred second condition (catalytic condition) is applied the first labelling moiety parts and second labelling moiety parts react to form the labelling moieties, which is a doublet molecule comprising a FRET donor and acceptor that are covalently conjugated. In this case the formation of said doublet molecule can be detected, because of the very high energy transfer efficiency, by means such as a microscope (emission, excitation, lifetime changes, acceptor photobleaching).
- **Affinity reagent-based detection:** The detection of the labelling moieties may also be performed or facilitated by using antibodies, nanobodies, aptamers, polymeric binders or small molecule binders that bind to the labelling moiety with high affinity and specificity, which are in turn labelled with a detectable label. In this case the labelling moiety may be a fluorescent dye or a non-fluorescent molecule.

Preferably, prior to and/or after applying the catalytic condition the biological sample is washed in order to remove markers not bound to a target analyte. In particular, this may reduce background noise.

Preferably, the first affinity reagent of the marker is configured to bind to a first target analyte of the biological sample and the second affinity reagent of the marker is configured to bind to a second target analyte of the biological sample, and wherein an optical property, in particular fluorescent intensity, of the marker is determined in the generated optical readout in order to determine a proximity, in particular a distance, between the first target analyte and the second target analyte. This enables efficiently determining the proximity or distance between the first target analyte and the second target analyte by means of an optical readout device.

Preferably, the first affinity reagent of the marker is configured to bind to a first binding site of a target analyte of the biological sample and the second affinity reagent of the marker is configured to bind to a second binding site of the target analyte of the biological sample. In particular, an optical property, in particular fluorescent intensity, of the marker is determined in the generated optical readout in order to determine the presence and/or location of the target analyte in the biological sample. This enables determining the presence and/or the location of the target analyte accurately.

Preferably, prior to generating the optical readout a secondary marker comprising an affinity reagent configured to specifically bind to the labelling moiety and a secondary label is introduced into the biological sample. This enables detecting the marker indirectly via the secondary marker.

Preferably, the secondary label is one of the following an enzyme, a metal tag, a fluorescent structure, a unique oligonucleotide sequence. This enables flexibly detecting the secondary marker. For example, the secondar label may be a fluorescent dye.

In a further aspect, a labelling kit is provided comprising a first label, the first label comprising a first polymeric backbone and a plurality of first labelling moiety parts attached to the first polymeric backbone, and a second label, the second label comprising a second polymeric backbone and a plurality of second labelling moiety parts attached to the second polymeric backbone. The first labelling moiety parts and the second labelling moiety parts are configured to bind to the respective other one to form labelling moieties. Further, the first polymeric backbone is configured to be attached to a first affinity reagent and the second polymeric backbone is configured to be attached to a second affinity reagent, in particular, in order to form a marker as described above.

The labelling kit and the method have the same advantages as the marker. Further, the labelling kit and the method may be supplemented with the features of the marker described in this document, in particular, the features of the dependent claims of the marker.

### Short Description of the Figures

Hereinafter, specific embodiments are described referring to the drawings, wherein:
- Figure 1: is a schematic view of a marker with a first marker part and a second marker part,
- Figure 2: is a schematic view of the marker according to Fig. 1 and an inactivation step,
- Figure 3: is a schematic view of the maker according to Fig. 1 with labelling moieties partially formed,
- Figure 4: is a schematic view of affinity reagents of another marker,
- Figure 5: is a schematic view of a further marker,
- Figure 6A: is a schematic view of a marker with short polymeric backbones,
- Figure 6B: is a schematic view of the marker according to Fig. 6A,
- Figure 7: is a schematic view of a marker with a coiled structure,
- Figure 8: is a schematic view of a marker with a multi-arm linker,
- Figure 9: is a schematic view of a marker with dendrimeric structures, and
- Figure 10: is a schematic view of a marker with a secondary marker.

### Detailed Description

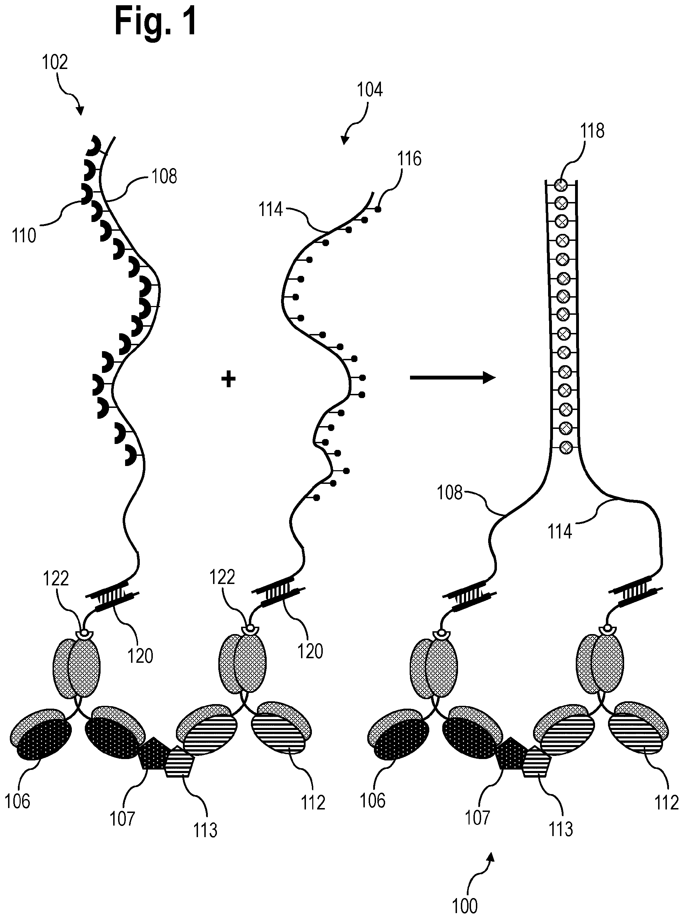

Figure 1 is a schematic view of a marker 100 with a first marker part 102 and a second marker part 104. The first marker part 102 and the second marker part 104 are configured to bind to each other in order to generate the marker 100.

The first marker part 102 comprises a first affinity reagent 106 configured to specifically bind to a first target analyte 107 of a biological sample (not shown). Further, the first marker part 102 comprises a first polymeric backbone 108 that is attached to the first affinity reagent 106. The first marker part 102 further comprises a plurality of first labelling moiety parts 110 that are attached to the first polymeric backbone 108.

Similarly, the second marker part 104 comprises a second affinity reagent 112 configured to specifically bind to a second target analyte 113 of the biological sample. Further, the second marker part 104 comprises a second polymeric backbone 114 that is attached to the second affinity reagent 112. The second marker part 104 further comprises a plurality of second labelling moiety parts 116 that are attached to the second polymeric backbone 114.

The affinity reagent 106, 112 are antibodies, for example. Alternatively, the affinity reagents 106, 112 may be an antibody fragment or an aptamer. The target analytes 107, 113 may be different proteins of the biological sample, for example.

The polymeric backbones 108, 114 may each comprise nucleic acids. Alternatively, the polymeric backbones 108, 114 may comprise polyethylene glycol. The polymeric backbones 108, 114 each extend essentially linearly in a direction away from the respective affinity reagent 106, 112. The labelling moiety parts 110, 116 are arranged along the respective polymeric backbone 108, 114. In particular, the labelling moiety parts 110, 116 are arranged in a single file in that direction along the respective polymeric backbone 108, 114.

The labelling moiety parts 110, 116 (as shown on the left side of Fig. 1) are each configured to bind to the respective other one forming labelling moieties 118. Thus, upon bringing the labelling moiety parts 110, 116 in close proximity, one of the first labelling moiety parts 110 may bind to one of the second labelling moiety parts 116 to generate one labelling moiety 118, for example. In particular, the binding of the labelling moiety parts 110, 116 results in the generation of the marker 100. In particular, the ratio of first labelling moiety parts 110 binding to second labelling moiety parts 116 is 1:1, thus one first labelling moiety part 110 binds to only one of the second labelling moiety parts 116 to generate one labelling moiety 118.

Preferably, the labelling moiety parts 110, 116 are each configured to bind to the respective other one only under a catalytic condition. This enables control over the formation of the marker 100 from the first and second marker parts 102, 104 depending on whether the catalytic condition is present. For example, the catalytic condition may be the presence of a particular catalyst. Examples of a suitable catalyst include UV-light or ions such as copper.

In a particular embodiment of the marker 100, the labelling moieties 118 may be optically detectable, whereas the labelling moiety parts 110, 116 are not optically detectable. For example, the labelling moieties 118 may be fluorophores that are each formed from a first labelling moiety part 110 and a second labelling moiety part 114.

The maker 100 may therefore indicate that the first target analyte 107 and the second target analyte 113 are in proximity, when the marker 100 forms from the marker parts 102, 104 (shown on the right side of the arrow of Fig. 1). Only then the marker 100, in particular the labelling moieties 118, is optically detectable, for example, by means of a microscope. In particular, the target analytes 107, 113 are in this case in such proximity that the labelling moiety parts 110, 116 may bind to each other.

The first and second affinity reagents 106, 112 may each comprise an attachment oligonucleotide 120. The attachment oligonucleotides 120 may be attached to the respective affinity reagent 106, 112 covalently, or via a biotin-streptavidin linker 122. The first and second polymeric backbones 108, 114 may be attached to the respective attachment oligonucleotide 120 via hybridisation. For example, each polymeric backbone 108, 114 may comprise an oligonucleotide with a sequence complementary to at least a part of the sequence of the respective attachment oligonucleotide 120. In particular, the first polymeric backbone 108 and the second polymeric backbone 114 and the respective attachment oligonucleotides 120 may have unique sequences that are complementary and that enable the specific attachment of the first and second polymeric backbones 108, 114 to the respective affinity reagent 106, 112. This enables efficient assembly of the marker parts 102, 104.

In case the marker 100 does not comprise attachment oligonucleotides 120, the polymeric backbones 108, 114 may be directly attached to the respective affinity reagents 106, 112.

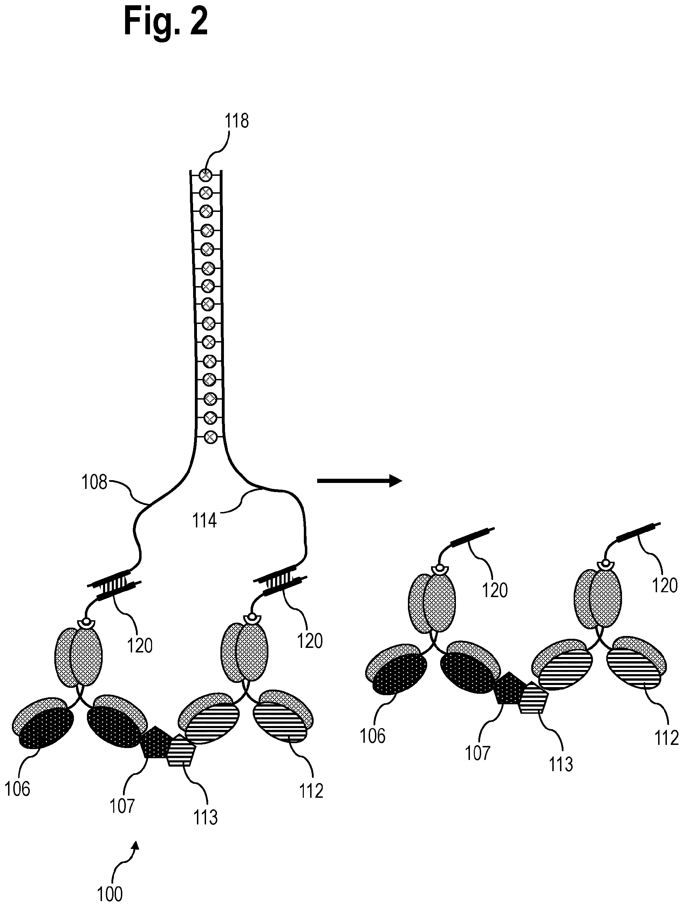

Figure 2 is a schematic view of the marker 100 (left side of Fig. 2) and an inactivation step of the marker 100 (right side of Fig. 2). The marker 100 may be inactivated by applying a degradation agent that degrades the polymeric backbones 108, 114 of the marker 100, for example. The degradation results in the removal or dissociation of the polymeric backbones 108, 114, in particular of the labelling moieties 118, from the marker 100. The attachment oligonucleotides 120 are preferably not removed from the respective affinity reagents 106, 112. Thus, further polymeric backbones 108, 114 may be attached to the attachment oligonucleotides 120 of the affinity reagents 106, 112. This enables iterative staining methods using the marker 100.

In case the marker 100 does not comprise the attachment oligonucleotides 120, application of the degradation agent may similarly result in inactivation of the marker 100 by removal of the polymeric backbones 108, 114. For example, a nuclease may be applied to degrade the polymeric backbones 108, 114, in case these comprise nucleic acids.

In a particular embodiment, the polymeric backbones 108, 114 may comprise a linker specifically susceptible to the degradation agent. For example, the polymeric backbones 108, 114 may comprise a cleavable linker, in particular arranged at or in the sequence complementary to the attachment oligonucleotide 120. Thus, when cleaving the linker, the complementary sequence is cleaved causing the polymeric backbones 108, 114 to dissociate from the attachment oligonucleotide 120.

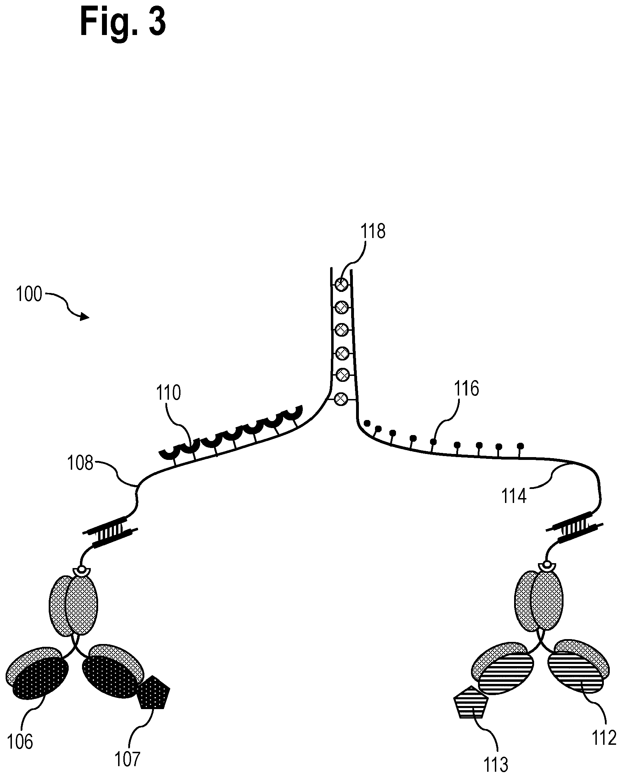

Figure 3 is a schematic view of the maker 100 with labelling moieties 118 partially formed from the first and second labelling moiety parts 110, 116. Thus, a proportion of the first and second labelling moiety parts 110, 116 are not in proximity to each other such that they would form labelling moieties 118. Whereas another proportion of first and second labelling moiety parts 110, 116 have already formed labelling moieties 118 since they are in close proximity.

In contrast to Fig. 1 and 2, the first target analyte 107 and the second target analyte 113 are located at a greater distance from each other (in particular, in the biological sample which is not shown) in Fig. 3. Consequently, the first and second marker parts 102, 104, in particular their respective affinity reagents 106, 112 are at a greater distance to each other. This results in only a proportion of the first and second labelling moiety parts 110, 116 forming labelling moieties 118. Thus, the degree to which labelling moieties 118 have formed from the first and second labelling moiety parts 110, 116 is proportional to the distance or proximity of the target analytes 107, 113.

In order to determine the proportion of the formed labelling moieties 118, an optical signal of the marker 100 may be determined, for example by means of a microscope. In case only the labelling moieties 118 are optically detectable fluorophores and the first and second labelling moiety parts 110, 116 are not optically detectable, the intensity of the fluorescence of the labelling moieties 118 may be an indicator of the proportion labelling moieties 118 formed and therefore the proximity or distance between the target analytes 107, 113.

In another case, at least one of the first and second labelling moiety parts 110, 116 are optically detectable and the labelling moieties 118 are not optically detectable. For example, one of the first or the second labelling moiety parts may be quenchers that quench the fluorescence of the other one of the first or the second labelling moiety parts may be fluorophores quenched by the quenchers. Thus, when the labelling moieties 118 forms, due to the quenching no optically detectable signal may be detected.

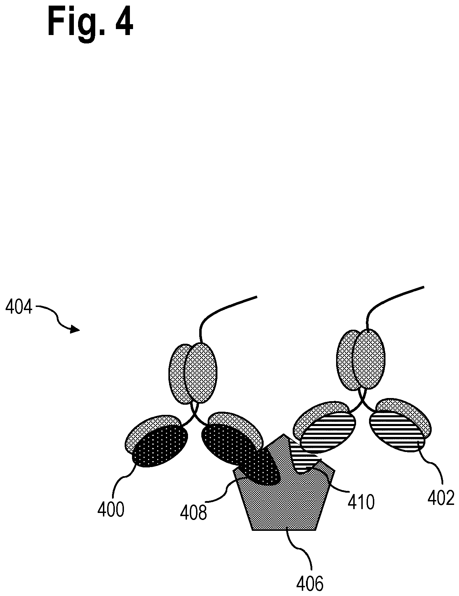

Figure 4 is a schematic view a first affinity reagent 400 and a second affinity reagent 402 of a marker 404. The first and second affinity reagent 400, 402 are configured to bind to the same target analyte 406, for example a protein. However, the first affinity reagent 400 is configured to specifically bind to a first part or epitope 408 of the target analyte 406. Similarly, the second affinity reagent 402 is configure to specifically bind to a second part or epitope 410 of the target analyte 406. Further aspects of the marker 404 are not shown in Fig. 4 for simplicity. Similarly to the marker 100 described above, the marker 404 may comprise marker parts, in particular the first and second polymeric backbones 108, 114 with the first and second labelling moiety parts 110, 116.

The marker 404 binding to the same target analyte 406 enables a reliable detection of the presence of the target analyte 406. For example, in case one or both of the affinity reagents 400, 402 also bind to other analytes, also called off-targets, the marker 404 with two affinity reagents 400, 402 reduces the likelihood of the marker 404, including both affinity reagents 400, 402, binding to an off-target. This is because an optical signal may be generated only when the marker parts of the marker 404 are in proximity, as described for the marker 100 above. Thus, the marker, and a corresponding method, may be used to mitigate the problem of cross-reactivity of affinity reagents and allow for detection of analytes with higher specificity. This is of particular importance in multi-plex or high-plex assays like for example, when the marker is used for plasma proteomics and or in spatial biology application like the interrogation of protein-protein interactions in tissue sections. Likewise, it is a key advantage of the marker in diagnostic tests, when closely related pathogens shall be reliably distinguished from each other to yield tests with very high specificity and consequently low false-positive rates.

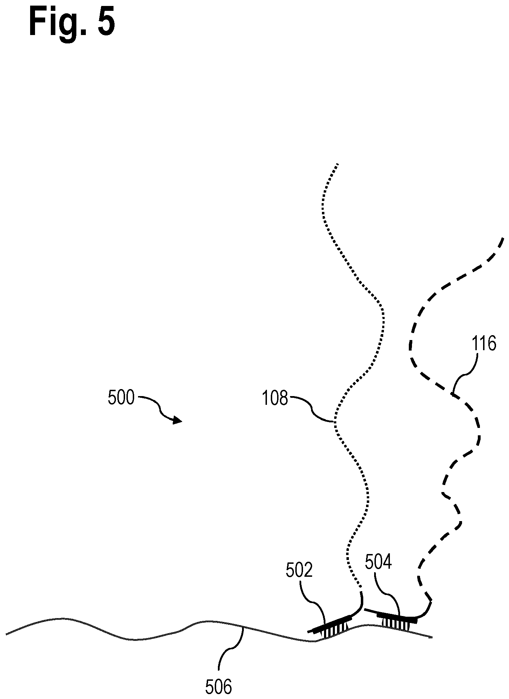

Figure 5 is a schematic view of a marker 500. The marker 500 comprises a first affinity reagent 502 and a second affinity reagent 504. The first and second affinity reagents 502, 504 are oligonucleotides configured to bind specifically to a nucleic acid target analyte 506. Thus, in contrast to the markers 100, 404 described above, the marker 500 may specifically bind to nucleic acid targets by hybridisation. The marker 500 may comprise marker parts including polymeric backbones 108, 116 and labelling moieties, as described above.

In a particular embodiment, a marker may comprise an amino acid based first affinity reagent, for example, the affinity reagent 400 and a nucleic acid based second affinity reagent, for example, the affinity reagent 504. In particular, this enables detecting an interaction between a protein and a nucleic acid target.

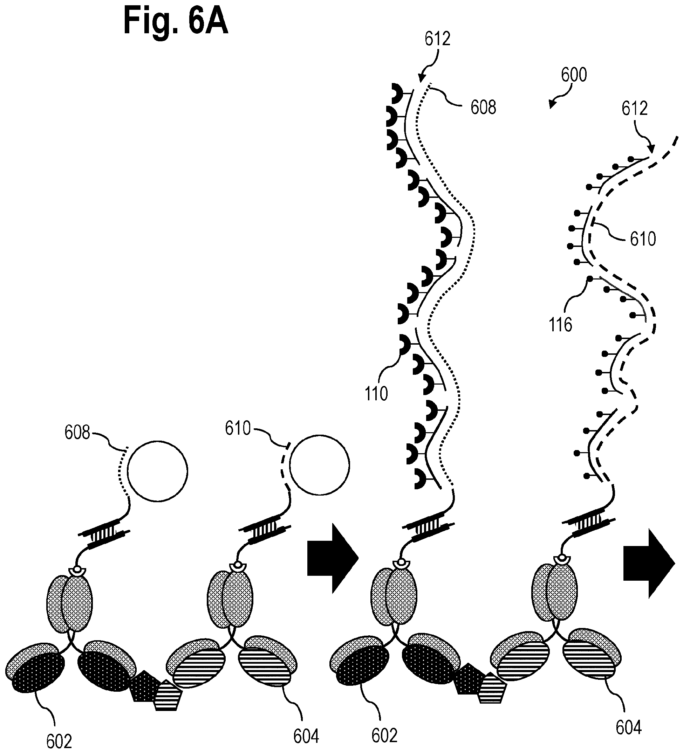

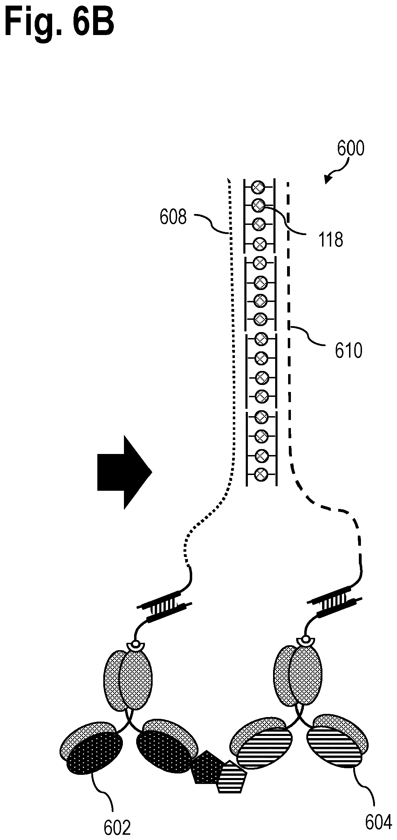

Figures 6A and 6B are schematic views of a marker 600 comprising a first affinity reagent 602 and a second affinity reagent 604, which comprise nucleic acid-based short polymeric backbones 608, 610. These may be conjugated to the first affinity reagent 602 and second affinity reagent 604 via hybridization, for example through barcoding which is advantageous in cases where multiple target analytes are analysed, or directly conjugated. The first labelling moiety parts 110 and the second labelling moiety parts 116 are in this case attached to the nucleic acid-based short polymeric backbones 608, 610 via hybridization 612 in a subsequent step. This provides numerous advantages: (1) the synthesis of the nucleic acid-based short polymeric backbones which are around 20-200nt in length is straightforward and inexpensive, (2) the synthesis of the oligonucleotides comprising the labelling moiety parts 110, 116 is likewise straightforward and inexpensive as their length can be kept in the range of 20-100nt, (3) this design offers the possibility to amplify the nucleic acid-based short polymeric backbones before introducing the oligonucleotides comprising the labelling moiety parts 110, 116. Such amplification may be performed using polymerase chain reaction (PCR), rolling circle amplification (RCA; as schematically depicted in the left view of Figure 6A), loop-mediated isothermal amplification (LAMP). Amplification may be optionally performed to boost the signal.

Following to the amplification, which extends the polymeric backbones 608, 610, the catalytic condition is applied and the first and second labelling moiety parts 110, 116 are allowed to react. This may form the labelling moieties 118, as shown in Figure 6B.

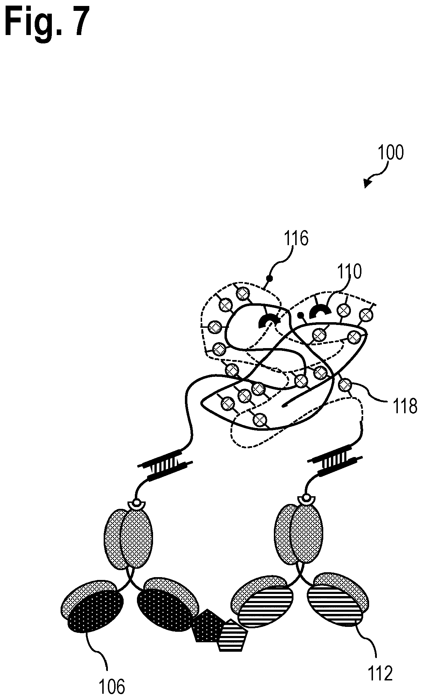

The formation of labelling moieties 118 may not in all cases proceed in the strictly ordered linear manner as depicted schematically in Figure 1, for example. In some cases, the reaction between the first labelling moiety parts 110 and the second labelling moiety parts 116 may lead to a coiled crosslinked structure, which may form a nanometer sized ball-like structure, for example. A schematic view of this possible behaviour is shown in Figure 7. As depicted in Figure 7, in such a situation there may be certain fraction of clickable groups, eg. first labelling moiety parts 110 and second labelling moiety parts 116 that cannot react with each other because of steric hindrance. While this will diminish the resulting signal, this is not a principal challenge to the method proposed here.

Nevertheless, there are ways in which an ordered assembly may be facilitated or even enforced. For example, when charged polymeric backbones like nucleic acids are used, an electrophoretic field may be applied to orient the polymeric backbones, in particular, prior to the reaction between the first labelling moiety parts 110 and the second labelling moiety parts 116 and, in particular, before the preferred addition of a catalyst. Furthermore, in the case of nucleic acid-based backbones staples strands may be provided that are configured to align or fold the polymeric backbones 108, 114 in a way that juxtaposes the first labelling moiety parts 110 and the second labelling moiety parts 116 in a regular arrangement before the catalytic condition is applied. As an example, suitable approaches are described in the application EP23174843.5.

Furthermore, it is conceivable to stretch the polymeric backbone108, 114 by applying a force through for example centrifugation or through the use of magnetic beads or magnetic particles and the application of a magnetic field, which can be attached to the ends of the polymeric backbones 108, 114.

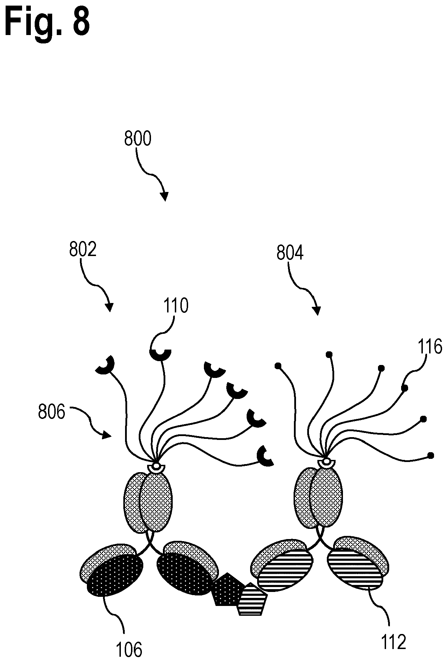

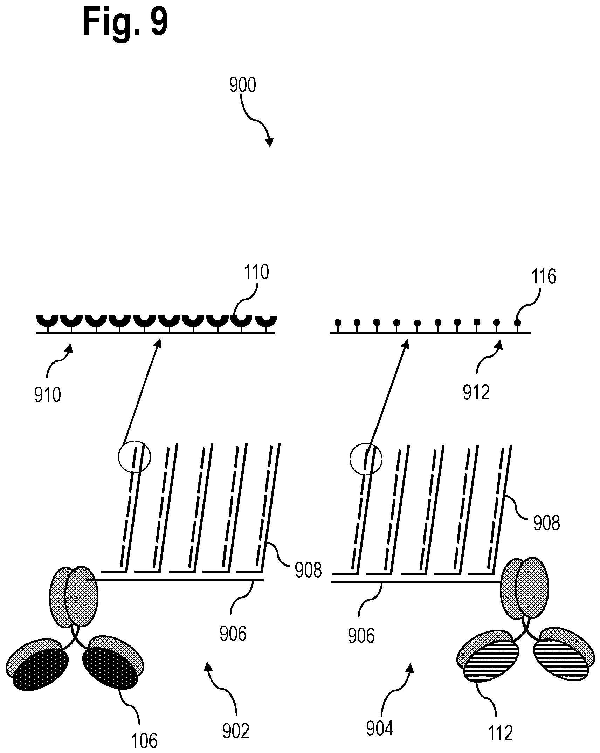

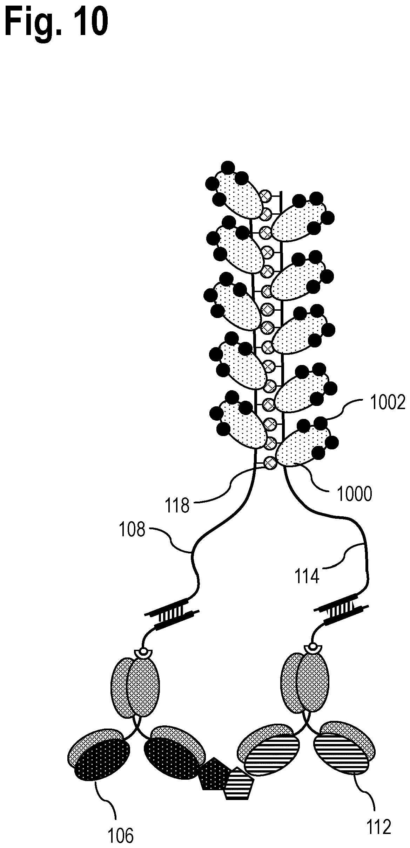

In cases where the detectable signal is an optical signal and low abundance targets or even single molecules shall be analysed or as well as in cases where the readout may be performed at high speeds, using low NA microscope objectives, or less sensitive, cheaper detectors/cameras, it may be desirable to amplify the signal in order to improve the signal detection. Several strategies exist to achieve signal amplification: (1) The amplification may affect the polymeric backbones 108, 114, as described in Figure 6A and 6B, (2) the amplification may result from using long polymeric backbones, (3) the amplification may result from using multiarm linkers schematically shown in Figure 8 like for example multiarm PEG-linkers, which can be generated with different valencies, functionalisations, and linker lengths, (4) the amplification may be achieved through dendrimeric structures, which may be based on hybridization and is schematically shown in Figure 9, (5) the amplification may be achieved through a secondary marker or secondary detection reagent that is used to detect the labelling moieties 118, as illustrated in Figure 10.

Figure 8 shows a marker 800 with a first marker part 802 and a second marker part 804, wherein the first labelling moiety parts 110 and the second labelling moiety parts 116 are multimerized on a multi-arm linker 806. Suitable linkers are for example 8-Arm PEG-Azide/Amine linkers, 8-Arm PEG-Azide/NHS, 8-Arm PEG-Alkyne/NHS linkers.

Figure 9 shows a marker 900 with a first marker part 902 and a second marker part 904, each marker part 902, 904 comprising a respective one of the affinity reagent 106, 112. The affinity reagents 106, 112 are each attached to a respective label comprising a nucleic acid polymeric backbone 906. In this example, each polymeric backbone 906 comprises hybridisation sites for five L-oligonucleotides 908, which serve as multimerization adapters. The L-oligonucleotides 908 may comprise for example another five hybridisation sites each for oligonucleotides 910 comprising the first labelling moiety parts 110 or for oligonucleotide 912 comprising the second labelling moiety parts 116. In this case a degree of labelling of 2-8 may be easily achieved for the oligonucleotides labelling of the antibody (e.g. the first affinity reagent 106 and the second affinity reagent 112). Thus for example, a single antibody 106, 112 may carry up to 8 x 5 x 5 x 10 = 2,000 labelling moiety parts 110, 116. As click reactions tend to yield very high efficiencies exceeding 80% a single marker configured in this may yield up to 1,600 labelling moieties 118. This enables a bright marker. Further, this number compares very favourably to the single molecule detection limit of a high-end confocal scanning microscope, which is in the range of 20-40 dye molecules. Even for optical readouts applying a microscope objective comprising a low NA with an inexpensive camera-based detection and a standard LED sample illumination, this number can be expected to yield a bright and easily detectable signal.

Figure 10 is a schematic showing the detection of the labelling moieties 118 using secondary markers comprising nanobodies 1000. Further, the secondary markers comprise detectable four dye molecules 1002 attached each nanobody 1000.

The markers 100, 404, 500, 600, 700, 900 described above may be applied in a method for analysing biological samples. Such biological samples may be for example: monolayer cell cultures, 3D cell cultures, spheroids, organoids, organ-on-a-chip and microphysiological systems samples, tissue biopsies (whole-mount) or tissue sections (cryosections, FFPE sections), cleared samples, water samples, environmental samples, as well as liquid biopsies and liquid samples prepared from any of the aforementioned.

Specifically, the markers may be introduced into the biological sample in a first step. Each marker may be specific to a particular target analyte or a set of two target analytes. Further, multiple copies of each marker may be introduced.

Subsequently, an optical readout of the biological sample with the markers may be generated. This enables detecting the target analytes in the biological sample, in particular, their presence or location in the biological sample.

In a preferred embodiment of the method, the markers 100, 404, 500 are introduced into the biological sample under a non-catalytic condition, which do not cause or enable the binding of the first and second labelling moiety parts 110, 116 to each other. In a subsequent step, the catalytic condition is applied to the markers 100, 404, 500. This enables formation of the labelling moieties 118, in case the first and second labelling moiety parts 110, 116 are in close proximity. In a subsequent step, the optical readout may be generated.

As described above, the optical readout may comprise optical signals from at least one of the first or second labelling moiety parts 110, 116 or the labelling moiety 118. This may be an indication of the presence of a single target analyte or a measure of the proximity of two target analytes.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100, 404, 500, 600, 800, 900: Marker
- 102, 104, 802, 804, 902, 904: Marker part
- 106, 112, 400, 402, 502, 504, 602, 604: Affinity reagent
- 107, 113, 406, 506: Target analyte
- 108, 114, 608, 610, 906: Polymeric backbone
- 110, 116: Labelling moiety part
- 118: Labelling moiety
- 120: Attachment oligonucleotide
- 122: Biotin-streptavidin linker
- 408, 410: Epitope
- 612: Hybridisation
- 806: Multi-arm linker
- 908: L-oligonucleotide
- 910,912: Oligonucleotide
- 1000: Nanobody
- 1002: Dye molecule

## Claims

1. A marker (100, 404, 500) for analysing a biological sample with a plurality of target analytes (107, 113, 406, 506) comprising:
a first marker part (102) comprising a first affinity reagent (106, 400, 502) and a first label, the first label comprising a first polymeric backbone (108) attached to the first affinity reagent (106, 400, 502) and a plurality of first labelling moiety parts (110) attached to the first polymeric backbone (108), and
a second marker part (104) comprising a second affinity reagent (112, 402, 504) and a second label, the second label comprising a second polymeric backbone (114) attached to the second affinity reagent (112, 402, 504) and a plurality of second labelling moiety parts (116) attached to the second polymeric backbone (114),
wherein the first labelling moiety parts (110) and the second labelling moiety parts (116) are configured to bind to the respective other one (110, 116) to form labelling moieties (118), and
wherein the first affinity reagent (106, 400, 502) and the second affinity reagent (112, 402, 504) are each configured to bind specifically to one of the target analytes (107, 113, 406, 506) of the biological sample.

2. The marker according to claim 1, wherein the first labelling moiety parts (110) and the second moiety parts (116) are configured to bind to each other under a catalytic condition.

3. The marker according to one of the preceding claims, wherein the first label is attached to the first affinity reagent (106, 400, 502) by a first cleavable linker and/or the second label is attached to the second affinity reagent (112, 402, 504) by a second cleavable linker.

4. The marker according to one of the preceding claims, wherein the first polymeric backbone (108) and/or the second polymeric backbone (114) comprises at least one nucleic acid.

5. The marker according to claim 4, wherein the first polymeric backbone (108) is attached to the first affinity reagent (106, 400, 502) by hybridisation and/or the second polymeric backbone (114) is attached to the second affinity reagent (112, 402, 504) by hybridisation.

6. The marker according to one of the preceding claims, wherein the first polymeric backbone (108) extends away from the first affinity reagent (106, 400, 502) in a first direction and the first labelling moiety parts (110) are arranged on the first polymeric backbone (108) along the first direction and/or the second polymeric backbone (114) extends away from the second affinity reagent (112, 402, 504) in a second direction and the second labelling moiety parts (116) are arranged on the second polymeric backbone (114) along the second direction.

7. The marker according to one of the preceding claims, wherein each first labelling moiety part (110) is equally spaced from any adjacent first labelling moiety part (110) and/or each second labelling moiety part (116) is equally spaced from any adjacent second labelling moiety part (116).

8. The marker according to one of the preceding claims, wherein the labelling moieties (118) are optically detectable.

9. The marker according to one of the preceding claims, wherein the first labelling moiety parts (110) and/or the second labelling moiety parts (116) are optically detectable.

10. The marker according to claim 9, wherein the first labelling moiety parts (110) and the second labelling moiety parts (116) differ in their optically detectable properties from the optically detectable property of the labelling moiety (118).

11. A method for analysing a biological sample comprising the steps:
introducing at least one marker (100, 404, 500) according to one of the preceding claims into the biological sample, and
generating an optical readout of the biological sample with the marker (100,, 404, 500).

12. The method according to claim 11, wherein during the step of introducing the marker (100, 404, 500), a non-catalytic condition is applied to the biological sample.

13. The method according to one of the preceding claims 11 or 12, wherein after the step of introducing the marker (100, 404, 500), a catalytic condition is applied to the biological sample.

14. The method according to one of the preceding claims 11 to 13, wherein the first affinity reagent (106) of the marker is configured to bind to a first target analyte (107) of the biological sample and the second affinity reagent (112) of the marker is configured to bind to a second target analyte (113) of the biological sample, and wherein an optical property of the marker is determined in the optical readout in order to determine a proximity between the first target analyte (107) and the second target analyte (113).

15. The method according to one of the preceding claims 11 to 13, wherein the first affinity reagent (400, 502) of the marker is configured to bind to a first binding site (408) of a target analyte (406, 506) of the biological sample and the second affinity reagent (402, 504) of the marker is configured to bind to a second binding site (410) of the target analyte (405, 506) of the biological sample.

16. The method according to one of the preceding claims 11 to 15, wherein prior to generating the optical readout a secondary marker comprising an affinity reagent (1000) configured to specifically bind to the labelling moiety (118) and a secondary label (1002) is introduced into the biological sample.

17. The method according to claim 16, wherein the secondary label (1002) is one of the following an enzyme, a metal tag, a fluorescent structure, a unique oligonucleotide sequence.

18. A labelling kit comprising:
a first label, the first label comprising a first polymeric backbone (108) and a plurality of first labelling moiety parts (110) attached to the first polymeric backbone (108), and
a second label, the second label comprising a second polymeric backbone (114) and a plurality of second labelling moiety parts (116) attached to the second polymeric backbone (114),
wherein the first labelling moiety parts (110) and the second labelling moiety parts (116) are configured to bind to the respective other one (110, 116) to form labelling moieties (118), and
wherein the first polymeric backbone (108) is configured to be attached to a first affinity reagent (106, 400, 502) and the second polymeric backbone (114) is configured to be attached to a second affinity reagent (106, 402, 504), in particular, in order to form a marker (100, 404, 500) according to one of the claims 1 to 10.
